# EUROPÄISCHE PATENTSCHRIFT

(11) **EP 3 727 599 B1**
(45) Veröffentlichungstag und Bekanntmachung des Hinweises auf die Patenterteilung: **22.03.2023**
(21) Anmeldenummer: 17851830.4
(22) Anmeldetag: 21.12.2017
(51) Int. Cl.: A61Q 5/10, A61K 8/34, A61K 8/41

(54) **MITTEL ZUM FÄRBEN VON KERATINFASERN ENTHALTEND EINE KOMBINATION AUS EINEM P-PHENYLENDERIVAT UND 2,7-NAPHTHALINDIOL SOWIE DESSEN VERWENDUNG ZUR REDUZIERUNG DES GELBSTICHS IN EINER FRISUR**
COMPOSITION FOR DYEING KERATIN FIBERS CONTAINING A COMBINATION OF A P-PHENYLENE DERIVATIVE AND 2,7-NAPHTHALENEDIOL AND USE THEREOF FOR REDUCING A YELLOW TINGE IN A HAIRSTYLE
AGENT DE COLORATION DE FIBRES DE KÉRATINE CONTENANT UNE COMBINAISON D'UN DÉRIVÉ DE P-PHÉNYLÈNE ET DE 2,7-NAPHTHALINE DIOL ET SON UTILISATION POUR DÉDUIRE LA DOMINANTE JAUNE DANS LA CHEVELURE

(43) Veröffentlichungstag der Anmeldung: 28.10.2020
(73) Patentinhaber: Laboratoire Biosthetique Kosmetik GmbH&Co. KG, 75179 Pforzheim (DE)
(72) Erfinder: BALZER, Wolfgang, 75179 PFORZHEIM (DE); AUGENSTEIN, Gunther, 75179 PFORZHEIM (DE); ADER, Christian, 75179 PFORZHEIM (DE)
(74) Vertreter: Keil & Schaafhausen Patentanwälte PartGmbB
(86) Internationale Anmeldenummer: PCT/EP2017/084021
(87) Internationale Veröffentlichungsnummer: WO 2019/120535

(56) Entgegenhaltungen:
- EP-A1- 1 671 619
- EP-A1- 1 707 242
- WO-A1-2015/181244
- DATABASE GNPD [Online] MINTEL; 1. November 2017 (2017-11-01), Schwarzkopf & Henkel: "Permanent Hair Colourant", XP002783696, Database accession no. 5246447
- DATABASE GNPD [Online] MINTEL; 1. April 2017 (2017-04-01), Procter & Gamble: "Hair Colourant Cream", XP002783697, Database accession no. 4775463
- DATABASE GNPD [Online] MINTEL; 1. Mai 2000 (2000-05-01), L'Oréal: "Grey Chic", XP002783698, Database accession no. 25981
- DATABASE GNPD [Online] MINTEL; 1. Februar 2012 (2012-02-01), Delly Kosmetic: "Anti-Yellowing Kit Treatment", XP002783699, Database accession no. 1738539

## Beschreibung

### Beschreibung

### Gebiet der Erfindung

Die vorliegende Erfindung betrifft Mittel zur oxidativen Färbung von Keratinfasern, insbesondere menschlichen Haaren, welche eine Kombination aus einem oder mehreren p-Phenylenderivaten als Entwicklersubstanzen und 2,7-Naphthalenediol als Kupplersubstanz enthalten. Neben menschlichen Haaren können auch tierische Haare, insbesondere Wolle oder Pelze, Federn, Nägel oder künstliche Keratinfasern gefärbt werden, wobei die vorliegende Erfindung insbesondere auf die Färbung von Kopfhaaren am Menschen gerichtet ist.

Die vorliegende Erfindung betrifft insbesondere auch die Verwendung des genannten Mittels zum Graufärben von menschlichem Kopfhaar, insbesondere von menschlichem Haar mit geringer Eigenpigmentkonzentration zur nachhaltigen Reduktion des Gelbstiches in einer Frisur die solche Haare enthält.

### Hintergrund der Erfindung

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 2-Methoxymethyl-1,4-p-Phenylendiamin, 1,4-Diaminobenzol und 4,5-Diamino-1-(2-hydroxyethyl)-pyrazol eingesetzt, während als Kupplersubstanzen beispielsweise Resorcin, 2-Methylresorcin, 1-Naphthol, 3-Aminophenol, 2-Amino-4-(2'-hydroxyethyl)amino-anisol und 1,3-Diamino-4-(2'-hydroxyethoxy)benzol zu nennen sind.

Darüber hinaus besteht auch die Möglichkeit, die Haare mit nichtoxidativen, direktziehende Farbstoffe enthaltenden Färbemitteln (häufig als Tönungsmittel bezeichnet) zu färben. Diese im folgenden Direktzieher genannten Farbstoffe besitzen nicht die Beständigkeit der oxidativ ins Haar eingebrachten Färbungen. Ein weiterer Vorteil der vorliegenden Erfindung liegt auch darin, dass solche Direktzieher ersatzlos entfallen können.

An Farbstoffe, die zur Färbung menschlicher Haare verwendet werden, sind neben der Stabilität der Haarfärbungen über einen bestimmten Zeitraum zahlreiche zusätzliche Anforderungen gestellt. So müssen die Farbstoffe in toxikologischer und dermatologischer Hinsicht unbedenklich sein und die erzielten Haarfärbungen sollen eine gute Lichtechtheit, Dauerwellechtheit, Reibeechtheit und Stabilität gegenüber anderen mechanischen Beanspruchungen und den üblichen Haarbehandlungen, insbesondere Schamponieren sowie eine ausreichende Beständigkeit gegenüber Hautabsonderungen wie z.B. Schweißabsonderungen aufweisen.

Ein besonderes Problem stellt bei der Nuancierung von Farbtönen die Herstellung natürlicher Grautöne dar, die zunehmend insbesondere zur Kaschierung eines Gelbstiches in der Frisur benötigt werden, welcher häufig bei natürlich ergrauten Haaren mit geringer Eigenpigmentkonzentration auftritt. Zur Erzielung natürlich wirkender grauer Nuancen ist es notwendig eine Vielzahl unterschiedlicher Oxidationsfarbstoffe oder Direktzieher zu kombinieren, die gemeinsam eine Nuancierung aus blauer, gelber oder roter Farben erfordert.

Auf dem Markt befindliche Produkte, die dieses Problem lösen sollen und die genannten Oxidationsfarbstoffe oder Direktzieher verwenden, liefern zwar nach der Färbung gleichmäßige, natürliche Grautöne, zeigen in Hinblick auf die Haltbarkeit aber gravierende Schwächen. Insbesondere zeigt sich in der Regel nach ca. 14 Tagen durch die Einwirkung von Licht, insbesondere UV-Licht oder durch das regelmäßige Schamponieren eine unerwünschte Veränderung der Nuancierung, da einzelne Farbkomponenten abgebaut oder ausgewaschen werden. Es wurde nunmehr überraschend gefunden, dass bei der Verwendung von einer oxidativen Kombination von 1,4-Diaminobenzol (p-Phenylendiamin) oder einem p-Phenylendiamin-derivat und 2,7-Naphthalenediol, wobei das 1,4-Diaminobenzol (p-Phenylendiamin) oder p-Phenylendiamin-derivat ausgewählt ist aus 1,4-Diamino-benzol (p-Phenylendiamin), 1,4-Diamino-2-methyl-benzol (p-Toluyldiamin), 1,4-Diamino-2-methoxymethyl-benzol, 1,4-Diamino-2-hydroxyethyl-benzol oder N,N-Bis-(2-Hydroxyethyl)-p-phenylendiamin, oder Gemischen daraus, die gewünschte Haltbarkeit bei der Erzielung natürlicher Grautöne sowie die Farbstabilität in besonders hohem Maße gewährleistet werden.

Die Verwendung von 2,7-Naphthalenediol als Kupplersubstanz, allerdings immer nur als Teil einer Mischung mit mehreren anderen Kupplern, ist zwar in der Literatur beschrieben, allerdings ohne Hinweis auf die Verbesserungen bei der Haltbarkeit und Farbstabilität von Grautönen.

Zum Beispiel wird in der EP 1 707 242 und der EP 1 671 619 A1 die Verwendung von 2,7- Dihydroxynaphthalin zusammen mit mindestens einem weiteren Kuppler erwähnt, allerdings nur in Bezug auf die Haltbarkeit von intensiven Farben ohne überhaupt Grautöne zu erwähnen oder in den Beispielen aufzuführen.

Das oxidative "Permanent Hair Colourant" von Schwarzkopf & Henkel zum Erzielen kühler, blonder Farbtöne enthält ebenfalls eine Kombination von 2,5-Diaminotoluolsulfat mit 2,7-Naphthalenediol und weiteren Kupplersubstanzen.

Andere oxidative Färbemittel zum Erzielen einer Graufärbung oder Verringerung eines Gelbstichs wie die Produkte "Hair Colourant Cream" von Procter & Gamble, "Grey Chic" von L'Oréal oder "Anti-Yellowing Kit Treatment" von Delly Kosmetic basieren zwar auf p-Phenylendiaminen, enthalten jedoch anstatt 2,7-Dinaphthalendiol eine Anzahl anderer Kuppler.

In WO 2015/181244 A1 werden Indol(in)-basierte Färbemittel beschrieben, welche neben der Indol(in)-Komponente, eine aromatische, polyzyklische Kohlenwasserstoffverbindung wie 2,7-Dinaphthalenediol sowie mindestens ein Übergangsmetall- und/oder Seltenerdmetallsalz enthalten, um den Farbdrift derartiger Färbemittel zu verhindern.

### Die vorliegende Erfindung

Gegenstand der vorliegenden Erfindung ist daher ein Mittel zur oxidativen Färbung von Keratinfasern, wie zum Beispiel von Wolle, Pelzen, Federn oder Haaren und insbesondere menschlichen Haaren, welches dadurch gekennzeichnet ist, dass es eine Kombination aus aus 1,4-Diaminobenzol (p-Phenylendiamin) oder einem p-Phenylendiamin-derivat als Entwickler, wobei das 1,4-Diaminobenzol (p-Phenylendiamin) oder p-Phenylenediamin-derivat ausgewählt ist aus 1,4-Diamino-benzol (p-Phenylendiamin), 1,4-Diamino-2-methyl-benzol (p-Toluyldiamin), 1,4-Diamino-2-methoxymethyl-benzol, 1,4-Diamino-2-hydroxyethyl-benzol oder N,N-Bis-(2-Hydroxyethyl)-p-phenylendiamin, oder Gemischen daraus, und 2,7-Naphthalenediol als Kuppler enthält. Des Weiteren richtet sich die gegenwärtige Erfindung auf eine Verwendung dieses Mittels zur Erzielung einer lange haltbaren Graufärbung von Haaren, insbesondere von bereits pigmentarmen, also ergrauten Haaren. Dabei wird die Gelbstichigkeit, die oft in Frisuren mit solchen Haaren auftritt, besonders nachhaltig kaschiert.

Das gebrauchsfertige Mittel zur oxidativen Färbung wird üblicherweise erst kurz vor der Anwendung zubereitet, zumindest was die Vermischung von Farbstoffkomponente und Oxidationsmittel angeht. Gegebenenfalls können auch andere Komponenten dann beigemischt werden, insbesondere wenn der Verwender entscheiden können soll, ob oder in welcher Dosierung sie verwendet werden sollen, wie z.B. für Rheologie beeinflussende Mittel. Natürlich macht dies eine zusätzliche Verpackung nötig. Alternativ können solche anderen Komponenten, aber auch in der Farbstoffkomponente oder in dem Oxidationsmittel enthalten sein, allerdings wären sie dann nicht mehr optional. Es hat sich als praktisch und daher üblich erwiesen die Farbstoffkomponente und das Oxidationsmittel, sowie weitere Komponenten gesondert verpackt dem Verbraucher oder Anwender zur Verfügung zu stellen.

Als Entwicklersubstanzen in der Farbstoffkomponente für das Färbemittel kommen dabei 1 ,4-Diamino-benzol (p-Phenylendiamin), 1 ,4-Diamino-2-methyl-benzol (p-Toluylendiamin), 1 ,4-Diamino-2- methoxymethyl-benzol, 1 ,4-Diamino-2-hydroxyethyl-benzol oder N,N-Bis- (2-Hydroxyethyl)-p-phenylendiamin in Betracht, wobei die Verbindungen in Form ihrer freien Basen oder Salze eingesetzt werden können.

Die Gesamtmenge an Entwickler- bzw. Kupplersubstanzen kann dabei in der erfindungsgemäßen Farbstoffkomponente etwa jeweils 0,05 bis 2 Gewichtsprozent der Farbstoffkomponente, insbesondere etwa 0,2 bis 1 Gewichtsprozent der Farbstoffkomponente betragen.

Darüber hinaus können in der Farbstoffkomponente des Färbemittels, falls diese zur Färbung von Haaren verwendet werden sollen, noch weitere übliche kosmetische Zusätze, beispielsweise Antioxidantien wie Ascorbinsäure, Thioglykol-säure oder Natriumsulfit, sowie Parfümöle, Komplexbildner, Netzmittel, Emulgatoren, Verdicker und Pflegestoffe enthalten sein.

Die Zubereitungsform der erfindungsgemäßen Farbstoffkomponente des Färbemittels kann beispielsweise eine Lösung, insbesondere eine wässrige oder wässrig-alkoholische Lösung, eine Paste, eine Creme, ein Pulver, ein Granulat, ein Gel, eine Emulsion, eine Dispersion, ein mit Luft erzeugter Schaum oder eine Aerosolzubereitung sein. Ihre Zusammensetzung stellt eine Mischung der Farbstoffkomponenten mit den für solche Zubereitungen üblichen Zusätzen dar.

Übliche Zusätze in Lösungen, Cremes, Emulsionen, Dispersionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent der Farbstoffkomponente, die Verdicker in einer Menge von etwa 0,1 bis 30 Gewichtsprozent der Farbstoffkomponente und die Pflegestoffe in einer Konzentration von etwa 0,1 bis 5,0 Gewichtsprozent der Farbstoffkomponente.

Geeignete Trägermaterialien für die Pulver- oder Granulat-Anwendungen sind pulvrige, mikrokristalline Substanzen die die Farbstoffe in einen physikalischen Zustand versetzen, der es erlaubt, das Verfahren zum Coaten der Pellets mit geeigneten Verkapselungsmaterialien durchzuführen. Geeignete Trägermaterialien sind insbesondere Gummi Arabicum, Dextrose, Polyvinylpyrrolidon, Oligosaccharide, mikrokristalline Cellulose-Derivate, wie zum Beispiel Hydroxypropylmethylcellulose, Methylcellulose, Hydroxyethyl-cellulose, Hydroxypropylcellulose, Nonoxynol-Hydroxyethyl-cellulose und Cetyl-Hydroxyethylcellulose oder physikalisch beziehungs-weise chemisch modifizierte Stärken oder Stärkederivate, wie zum Beispiel Stärkeester (beispielsweise acetylierte Stärken), Stärkeether (beispielsweise hydroxyalkylierte Stärken), Dialdehydstärken, Dicarboxylstärken, Distärkephosphate, Hydroxyalkylstärkephosphate oder Hydroxyalkylstärken, wobei die Alkylgruppen vorzugsweise 1 bis 4, besonders bevorzugt 2 bis 3 C-Atome enthalten. Geeignet sind auch quervernetzte Stärkeether, wie zum Beispiel solche mit den INCI-Bezeichnungen Dimethylimidazolidone Rice beziehungsweise Corn Starch oder hydrophob modifizierte Stärken (beispielsweise solche mit der INCI-Bezeichnung Aluminium Starch Octensuccinate). Die Stärke kann sowohl thermisch als auch hydrolytisch oder enzymatisch modifiziert worden sein, wobei die Ausgangsstärke aus den bekannten Quellen, beispielsweise Mais, Kartoffeln, Süsskartoffeln, Erbsen, Bananen, Hafer, Weizen, Gerste, Reis, Sago, Tapioca, Pfeilwurz, Amarant, Kanna, Sorghum, usw., gewonnen werden kann. Besonders bevorzugte Stärkederivate sind nichtionische Stärkederivate, insbesondere mit Alkylenoxiden wie Ethylenoxid, Propylenoxid oder Butylenoxid, Acetanhydrid oder Butylketendimer, und insbesondere Propylenoxid, modifizierte nichtionische Stärkederivate. Weitere geeignete Trägermaterialien sind synthetisches Calciumsilicat, Kieselgur, Siliziumdioxid oder andere freifließende, nicht zusammenbackende Pulver.

Geeignete Verkapselungsmaterialien für die Pulver oder Granulate sind wasserlösliche oder wasserdispergierbare, filmbildende Substanzen, die in der Lage sind, aus Lösungen oder Dispersionen, sich durch Sprühtrocknung derart auf den Pulvern einheitliche Filme abzuscheiden, dass von einer Ummantelung (Coating) gesprochen werden kann. Geeignete Verkapselungsmaterialien sind Gummi Arabicum, CelluloseDerivate (beispielsweise Methylcellulosen), Polyethylen-Dispersionen, Polyacrylsäuren, Polyvinylalkohole, Polycarbonate, Polyvinylpyrrolidon, Polyester und Polyamide oder natürliche Filmbildner wie zum Beispiel Chitosan, Schellack, Oligosaccharide oder auch chinesisches Balsamharz (Kolophonium).

Eine Entstaubung der pulverförmigen Zusammensetzungen kann auch durch eine aus Ölen und Wachsen, beispielsweise einer organisch lipophilen Verbindung aus der Gruppe der pflanzlichen und/oder tierischen Fette, Öle und Wachse, der Paraffinkohlenwasserstoffe, der höheren Alkohole und Ether, der aliphatischen und aromatischen Ester sowie der Silikonöle, bestehenden hydrophoben Basis erreicht werden.

Ebenso ist es möglich Suspensionen auszubilden, welche zusätzlich mindestens einen anorganischen oder organischen Verdicker mit lipophilem Charakter, der mit der lipophilen Verbindung ein Oleogel bzw. Lipogel ausbildet, enthalten können.

Als organisch lipophile Verbindungen eignen sich insbesondere Pflanzenöle wie zum Beispiel Jojobaöl; Vaseline; flüssige Paraffine, beispielsweise Paraffinum Perliquidum und Paraffinum Subliquidum; Siliconöle; flüssige, langkettige, hydrophobe Fettsäureester, beispielsweise Octylpalmitat, Isocetylpalmitat, Isopropylpalmitat und Octylstearat; wachsförmige, langkettige, hydrophobe Fettsäureester und/oder synthetische Wachsersatzstoffe, beispielsweise natürliches oder synthetisches Bienenwachs (zum Beispiel Lipowachs 6138G^{®} der Firma Lipo Chemicals), C18- bis C36-Fettsäuren (zum Beispiel Synchrowachs AW1C^{®} der Firma Croda Chemicals Ltd.), C-10 bis C36-Fettsäuretriglyceride, wie zum Beispiel Octansäure/Dodecansäure-Triglycerid, hydrierte Kokosfettsäureglyceride (zum Beispiel Softisan 100^{®} der Firma Hüls AG), Glyceryltribehenat (beispielsweise Synchrowachs HRC^{®} der Firma Croda Chemicals Ltd.), Fettsäureestergemische (beispielsweise Cutina BW^{®} der Firma Henkel KGaA), sowie deren Mischungen. Besonders bevorzugt ist hierbei die Verwendung von Jojobaöl, Fettsäureestern, Paraffinöl, Kombinationen aus Fettsäureestern und Paraffinöl sowie Kombinationen aus Fettsäureestern und/oder Paraffinöl mit Vaseline.

Die lipophilen Verbindungen werden, bezogen auf die Gesamtmenge der Farbkomponente, in einer Konzentration von etwa 0,1 bis 80 Gewichtsprozent, vorzugsweise von 3 bis 65 Gewichtsprozent, und insbesondere von 20 bis 50 Gewichtsprozent, eingesetzt.

Als lipophile anorganische oder organische Verdicker sind insbesondere Alkalicarboxylate, Erdalkalicarboxylate oder Aluminiumcarboxylate, beispielsweise Natriumpalmitat, Aluminium/Magnesiumhydroxystearat oder Magnesiumstearat, Aluminiummonostearat, Aluminiummonodistearat und/oder Aluminiumtristearat; Copolymerisate von Alkenen, wie zum Beispiel Ethylen/Propylen-Copolymere; vernetzte organische Polymere und lipophilisierte Schichtsilikate, wie zum Beispiel Benzyl-dimethylstearylammonium-Hectorit (beispielsweise Bentone 28 der Fa. Rheox), sowie Gemische dieser lipophilen Verdicker zu nennen, wobei Alkalistearate, Erdalkalistearate, Aluminiumstearate und Aluminium/ Magnesiumhydroxystearat, und insbesondere Magnesiumstearate und Aluminiumstearate, bevorzugt sind.

Je nach Zusammensetzung kann das erfindungsgemäße Färbemittel schwach sauer, neutral oder alkalisch reagieren.

Gemäß der vorliegenden Erfindung können die Färbemittel gegebenenfalls weiterhin mindestens eine Quelle für ein Alkalisierungsmittel, vorzugsweise eine Quelle für Ammoniak oder Ammoniumionen, enthalten. Als weitere Alkalisierungsmittel können bekannte Verbindungen, beispielsweise Alkanolamide wie zum Beispiel Monoethanolamin, Diethanolamin, Triethanolamin, Monopropanolamin, Dipropanolamin, Tripropanolamin, 2-Amino-2-methyl-1, 3-propandiol, 2-Amino-2-methyl-1-propanol, Tris(Hydroxymethyl)aminomethan oder 2-Amino-2-hydroxymethyl-1,3-propandiol verwendet werden. Besonders bevorzugte Alkalisierungsmittel sind solche Mittel, welche eine Quelle für Ammoniumionen aufweisen, wobei jede Quelle für Ammoniumionen geeignet ist. Bevorzugte Quellen für Ammoniumionen sind Ammoniumchlorid, Ammoniumsulfat, Ammoniumnitrat, Ammoniumphosphat, Ammoniumacetat, Ammoniumcarbonat, Ammoniumhydrogencarbonat, Ammoniumcarbamat, Ammoniumhydroxid, Percarbonatsalze, Ammoniak und deren Mischungen. Besonders bevorzugt sind hierbei Ammoniumcarbonat, Ammoniumcarbamat, Ammoniumhydrogencarbonat, Ammoniak und deren Mischungen. Die Mittel gemäß der vorliegenden Erfindung können etwa 0,1 bis etwa 10 Gewichtsprozent, vorzugsweise etwa 0,5 bis etwa 5 Gewichtsprozent, und insbesondere etwa 1 bis etwa 3 Gewichtsprozent eines Alkalisierungsmittels, vorzugsweise Ammoniumionen, enthalten.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht.

Gemäß der vorliegenden Erfindung enthalten die gebrauchsfertigen Färbemittel etwa 0,1 bis etwa 12 Gewichtsprozent, vorzugsweise etwa 0,5 bis 10 Gewichtsprozent, und insbesondere etwa 1 bis etwa 4 Gewichtsprozent des Oxidationsmittels.

Für die Anwendung mit Oxidationsmitteln vermischt man das vorstehend beschriebene Färbemittel unmittelbar vor dem Gebrauch mit dem Oxidationsmittel und trägt eine für die Haarfärbebehandlung ausreichende Menge, je nach Haarfülle, im Allgemeinen etwa 60 bis 200 Gramm, dieses Gemisches auf das Haar auf. Das Gewichtsverhältnis zwischen Haarfärbemittel und Oxidationsmittel kann dabei 5:1 bis 1:3, vorzugsweise jedoch 1:1 bis 1:2 betragen. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet.

Der pH-Wert des gebrauchsfertigen Färbemittels ist vorzugsweise gleich 4 bis 11 und stellt sich beim Vermischen des Färbemittels mit dem Oxidationsmittel ein. Gegebenenfalls kann der pH-Wert durch Zusatz von geeigneten Säuren oder Basen eingestellt werden.

Man lässt das Gemisch bei 15 bis 50 °C etwa 3 bis 60 Minuten lang, vorzugsweise 30 bis 45 Minuten lang, auf das Haar einwirken, spült sodann das Haar mit Wasser aus und trocknet es. Gegebenenfalls wird im Anschluss an diese Spülung mit einem Shampoo gewaschen und falls erforderlich mit einer schwachen organischen Säure, wie zum Beispiel Zitronensäure oder Weinsäure, nachgespült. Anschließend wird das Haar getrocknet.

Das erfindungsgemäße Färbemittel, welches eine Kombination aus 1,4-Diaminobenzol (p-Phenylendiamin) oder einem p-Phenylendiamin-derivat und 2,7-Naphthalenediol enthält, wobei das 1,4-Diaminobenzol (p-Phenylendiamin) oder p-Phenylendiamin-derviat ausgewählt ist aus 1,4-Diamino-benzol (p-Phenylendiamin), 1,4-Diamino-2-methyl-benzol (p-Toluyldiamin), 1,4-Diamino-2-methoxymethyl-benzol, 1,4-Diamino-2-hydroxyethyl-benzol oder N,N-Bis-(2-Hydroxyethyl)-p-phenylendiamin, oder Gemischen daraus, erfüllt die gestellten Anforderungen an die Erzielung natürlicher Grautöne, insbesondere in Bezug auf die Haltbarkeit bzw. Farbstabilität in besonders hohem Maße.

Ein weiterer Aspekt der vorliegenden Erfindung betrifft die Verwendung von den erfindungsgemäßen Färbemitteln bei der Herstellung lange haltbarer, natürlicher Grautöne zur Kaschierung eines Gelbstiches in einer Frisur. Solche Gelbstiche treten üblicherweise bei ergrautem Haar auf, das nur noch wenig eigene Pigmente enthält, insbesondere wenn die Eigenpigmentfarbe ursprünglich im dunkel blond bis schwarzem Bereich war. Natürlich kann solches Haar in jeder üblichen Nuance gefärbt werden, enthält dann aber wieder eine hohe Pigmentkonzentration, wobei sich wenig eigenes Pigment mit viel künstlichem Farbstoffpigmenten paart. Darauf zielen die Ansprüche der gegenwärtigen Verwendung jedoch nicht ab.

Die gegenwärtige Verwendung ist daher gerichtet auf die Graufärbung von ergrautem, leicht gelbstichigem Haar, dessen natürliche dunkle Haarfarbe im Bereich von dunkelblond bis schwarz lag und in dem der Anteil weisser, d.h. substanziell nicht-pigmentierter Haare größer als 10%, vorzugsweise größer als 50% und oft über 85% ist, jedoch 100% nicht erreicht. Der Grauanteil der Haare kann durch den Fachmann, den geschulten-Friseur, optisch analysiert oder im Labor mit Hilfe von Bildanalysesystemen bestimmt werden. Dabei wird die Anzahl der nicht mehr pigmentierten Haare in Bezug zu allen Haaren gesetzt und in Prozent angegeben. Eine dunkle Haarfarbe der natürlichen Haare kann durch den Fachmann, den geschulten-Friseur, optisch bestimmt werden oder im Labor mit Hilfe der Euronorm EN ISO 11664-4"Colorimetry -- Part 4: CIE 1976 L*a*b* Colour space" definiert werden. Solche Haare haben typischerweise Lab-Werte die in den folgenden Bereichen liegen: L kleiner als 30, a zwischen 0 und 5 und b zwischen 0 und 7.

Alle in der vorliegenden Anmeldung enthaltenen Prozentangaben stellen soweit nicht anders angegeben Gewichtsprozente dar.

Die nachfolgenden Beispiele sollen den Gegenstand der Erfindung näher erläutern, ohne ihn darauf zu beschränken.

### Beispiele

### Beispiele 1 bis 5: Haarfärbelösung

| | |
|---|---|
| x g | 1,4-Diaminobenzol oder p-Phenylendiamin-derivat |
| 0,44 g | 2,7-Naphthalenediol |
| 10,0 g | Kaliumoleat (8%ige wässrige Lösung) |
| 4,25 g | Monoethanolamin (100%ig) |
| 10,0 g | Ethanol |
| 0,3 g | Ascorbinsäure |
| ad 100,0 g | Wasser |

30 g der vorstehenden Haarfärbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 1 zusammengefasst.

**Tabelle 1: Farbergebnisse aus Färbelösung**

| **Beispiel** | **p-Phenylenderivat** | **Konz. (x)** | **Farbergebnis** |
|---|---|---|---|
| 1 | 1,4-Diamino-benzol | 0,295 g | grau (leicht violett) |
| 2 | 1,4-Diamino-2-methyl-benzol Sulfate | 0,600 g | stahlgrau |
| 3 | 1,4-Diamino-2-methoxymethyl-benzol | 0,415 g | grau (leicht rötlich) |
| 4 | 1,4-Diamino-2-hydroxyethyl-benzol | 0,683 g | grau (leicht rötlich) |
| 5 | N,N-Bis-(2-Hydroxyethyl)-p-phenylendiamin Sulfate | 0,852 g | grau (leicht türkis) |

### Beispiele 6 bis 8: Farbstabilitäten

### Erfindungsgemäßes Produkt (EP)

| | |
|---|---|
| 0,600 g | 1,4-Diaminobenzol oder p-Phenylendiamin-derivat |
| 0,44 g | 2,7-Naphthalenediol |
| 15,0 g | Cetylalkohol |
| 0,3 g | Ascorbinsäure |
| 3,5 g | Natriumlaurylalkoholdiglycolethersulfat, 28%ige wässrige Lösung |
| 3,0 g | Ammoniak, 22%ige wässrige Lösung |
| 0,3 g | Natriumsulfit, wasserfrei |
| ad 100,0 g | Wasser |

30 g der vorstehenden Haarfärbecreme werden unmittelbar vor der Anwendung mit 30 g einer 6-prozentigen Wasserstoffperoxidlösung vermischt. Anschließend wird das Gemisch auf Haare aufgetragen. Nach einer Einwirkzeit von 20 Minuten wird das Haar mit Wasser gespült, mit einem handelsüblichen Shampoo gewaschen und getrocknet.

Zum Vergleich werden drei Marktprodukte aus der Reihe IGORA ABSOLUTES SILVERWHITE, der Firma Henkel aus Düsseldorf, Deutschland laut Gebrauchsanweisung ebenfalls auf Haaren ausgefärbt:
Marktprodukt 1 (MP1 ): Grey Lilac
Farbstoffe laut INCI sind als Entwickler: 1 ,4-Diamino-2-methyl-benzol Sulfate, N,N-Bis-(2-Hydroxyethyl)-p-phenylendiamin Sulfate, und als Kuppler: Resorcinol, 4-Chlorresorcinol, 2-Methylresorcinol, m- Aminophenol, 4-Amino-2-hydroxytoluene
Marktprodukt 2 (MP2): Dove Grey
Farbstoffe laut INCI sind als Entwickler: 1 ,4-Diamino-2-methyl-benzol Sulfate, N,N-Bis-(2-Hydroxyethyl)-p-phenylendiamin Sulfate, und als Kuppler: Resorcinol, m-Aminophenol, 2,7-Naphthalenediol
Marktprodukt 3 (MP3): Slate Grey
Farbstoffe laut INCI sind als Entwickler: 1,4-Diamino-2-methyl-benzol Sulfate, N,N-Bis-(2-Hydroxyethyl)-p-phenylendiamin Sulfate, und als Kuppler: Resorcinol, m-Aminophenol, 2,7-Naphthalenediol.

### Überprüfung der Lichtstabilität

Von den mit den vier Produkten (EP, MP1, MP2, MP3) behandelten Strähnen (Yak-Haar) wurden die Lab-Meßwerte bestimmt. Anschließend wurden die Strähnen für eine Zeitdauer von drei Tagen dem Sonnenlicht ausgesetzt. Nach dieser Zeit wurden erneut die Lab-Werte gemessen. Die Ergebnisse sind in Tabelle 2 dargestellt.

### Überprüfung der Waschstabilität

Von den mit den vier Produkten (EP, MP1, MP2, MP3) behandelten Strähnen (gebleichte, blonde Naturhaare) wurden die Lab-Meßwerte bestimmt. Anschließend wurden die Strähnen für eine Zeitdauer von 24 h in eine 20 prozentige Shampoolösung gegeben. Anschließend wurden die Strähnen mit Wasser ausgespült und getrocknet. Danach wurden erneut die Lab-Werte gemessen.

Die Ergebnisse sind in Tabelle 3 dargestellt.

### Überprüfung der Tragestabilität am Modell

An einem Modell (Ausgangshaarfarbe mittelblond, Grauanteil 80-90 %) wurde im Halbseitentest die erfindungsgemäße Rezeptur gegen die Rezeptur MP 3 verglichen. Nach der Anwendung laut Gebrauchsanweisung wurde jeweils an 10 Stellen die Lab-Werte gemessen und ein Mittelwert gebildet.

Nach 14 Tagen wurden die Werte erneut gemessen.

Die Ergebnisse sind in Tabelle 4 dargestellt.

**Tabelle 2: Farbstabilität nach Lichteinwirkung**

| | Ausgangswerte | | | Werte nach Behandlung | | | | | | |
|---|---|---|---|---|---|---|---|---|---|---|
| Produkt | L | a | b | L | a | b | Δ L | Δ a | Δ b | Δ E |
| EP | 33,86 | 0,06 | 0,77 | 35,38 | 1,90 | 2,52 | 1,52 | 1,84 | 1,75 | 2,96 |
| MP1 | 39,72 | 1,33 | -4,10 | 44,37 | 0,92 | 0,24 | 4,65 | -0,41 | 4,34 | 6,37 |
| MP2 | 41,60 | -1,60 | -2,66 | 42,57 | -1,08 | 3,09 | 0,97 | 0,52 | 5,75 | 5,85 |
| MP3 | 30,65 | 1,30 | -2,15 | 27,09 | 0,70 | 1,10 | -3,56 | -0,60 | 3,25 | 4,86 |

Das Ergebnis zeigt, dass die erfindungsgemäße Rezeptur (EP) eine wesentlich geringere Gesamtfarbabweichung (Δ E) und somit eine bessere Stabilität gegenüber Lichteinwirkung besitzt.

**Tabelle 3: Farbstabilität nach Shampooeinwirkung**

| | Ausgangswerte | Werte nach Behandlung | | | | | | | | |
|---|---|---|---|---|---|---|---|---|---|---|
| Produkt | L | a | b | L | a | b | Δ L | Δ a | Δ b | Δ E |
| EP | 23,17 | 4,36 | -2,82 | 23,74 | 4,86 | -3,74 | 0,57 | 0,50 | -0,92 | 1,19 |
| MP1 | 35,64 | 1,25 | -0,66 | 42,42 | 1,16 | 0,23 | 3,78 | -0,09 | 0,89 | 6,84 |
| MP2 | 39,00 | 1,56 | 2,25 | 43,66 | 2,82 | 2,39 | 4,66 | 1,26 | 0,15 | 4,83 |
| MP3 | 2,775 | 2,07 | 1,35 | 36,66 | 2,41 | 3,91 | 4,35 | 0,08 | 0,51 | 4,38 |

Das Ergebnis zeigt, dass die erfindungsgemäße Rezeptur (EP) eine wesentlich geringere Gesamtfarbabweichung (Δ E) und somit eine bessere Stabilität gegenüber Shampooeinwirkung besitzt.

**Tabelle 4: Farbstabilität am Modell**

| | Ausgangswerte | | | Werte nach Behandlung | | | | | | |
|---|---|---|---|---|---|---|---|---|---|---|
| Produkt | L | a | b | L | a | b | L | a | b | E |
| EP | 46,36 | 3,02 | 6,23 | 46,15 | 3,78 | 9,76 | -0,21 | 0,76 | 3,53 | 3,61 |
| MP3 | 60,57 | 3,00 | 7,34 | 52,63 | 2,89 | 8,34 | -7,94 | -0,10 | 1,00 | 8,00 |

### Friseur-technische Bewertung der Erfindung

Zur Friseur-technischen Bewertung werden 2 Modelle jeweils mit erfinderischer Graufärbung und einem Marktprodukt behandelt. Die dabei übliche halbseitige Behandlung war nicht möglich, da der Unterschied des Resultates so signifikant und leicht erkennbar war, dass geeignete Modelle dafür nicht zur Verfügung standen, bis eine Vergleichsfärbung in einem reduzierten Teilbereich auf dem Kopf für das Marktprodukt (in diesem Fall das oben erwähnte MP3) angeboten wurde. Die daraus resultierenden Ergebnisse sind also keine klassischen Halb-Kopf Vergleiche, sondern Vergleiche zwischen einer Ganz-Kopf Behandlung mit dem erfinderischen Färbemittel, aber unter Aussparung einer Teilfläche auf dem Kopf und der Behandlung mit MP3 in dieser Teilfläche.

Die Friseur technische Beurteilung erfolgte jeweils durch 3 erfahrene Friseure mit über 10 jähriger Erfahrung in Färbearbeiten mit Test Modellen und den üblichen Beurteilungstechniken von Haarfarbergebnissen.

### Haarzustand vor der Färbung

**Modell H:** Naturton des Haares 5/0, Mittelbraun, Weißanteil an der Kontur 100%, am Oberkopf 50% und am Hinterkopf 30%. Haarlänge bis 30 cm. Ausschnitt für MP3 Vergleichsfärbung am Hinterkopf. Eine sehr starke und deutlich erkennbare Gelbstichigkeit der Haare wurde durch alle Friseure bestätigt und war besonders im Weißanteil der Haare auffällig.

**Modell Z:** Naturton des Haares 4/0, Mittelbraun, Weißanteil an der Kontur 100%, am Oberkopf 25% und am Hinterkopf 20%. Haarlänge bis 25 cm. Ausschnitt für MP3 Vergleichsfärbung auch am Hinterkopf. Eine starke und klar erkennbare Gelbstichigkeit der Haare wurde durch alle Friseure bestätigt, besonders im Weißanteils der Haare, was durch den im Vergleich mit Modell H geringeren Weißanteil weniger auffällig wirkte.

### Färbemittel Anwendung

Zur erfindungsgemäßen Färbung wird die unter EP genannte Haarfarbcreme wie unten angegeben zubereitet und verwendet, während zum Vergleich die Haarfarbcreme aus dem Marktprodukt MP3 zubereitet gemäß Gebrauchsanleitung verwendet wird.

Zur Zubereitung des verwendungsbereiten Färbemittels wird die EP Creme im Verhältnis 1 zu 4 mit einer 1,9%igen Peroxyd Lösung verdünnt und gemischt. Das resultierende Färbemittel wird mit einem Pinsel auf dem Haar aufgebracht und 20 Minuten einwirken gelassen, danach mit Wasser gespült, mit einem handelsüblichen Shampoo gewaschen und getrocknet.

### Bewertung und Ergebnisse:

Die Bewertung folgt dem deutschen Schulnotenprinzip, mit 1 als sehr gut, 2 als gut, 3 als befriedigend, 4 als ausreichend, 5 als mangelhaft und 6 als ungenügend. Die Bewertung durch die Friseure bezieht sich auf die Qualität des Färbeergebnisses einschließlich der empfundenen Reduktion oder Elimination eines vor Färbung klar hervortretenden Gelbstiches.

**Bewertung der erfindungsgemäßen Färbung bei Modell H**

| Friseur | Vor der Färbung | Tag der Färbung | Nach 7 Tagen | Nach 14 Tagen | Nach 21 Tagen | Nach 28 Tagen |
|---|---|---|---|---|---|---|
| Frau L. | 4,5 | 2,0 | 2,0 | 2,0 | 2,0 | 2,5 |
| Herr B. | 4,5 | 2,5 | 2,5 | 2,5 | 3,0 | 3,0 |
| Herr H. | 4,3 | 2,0 | 2,0 | 2,5 | 2,5 | 2,5 |
| Durchschn itt | 4,5 | 2,1 | 2,1 | 2,3 | 2,5 | 2,7 |

**Bewertung der Vergleichsfärbung bei Modell H**

| | Vor der Färbung | Tag der Färbung | Nach 7 Tagen | Nach 14 Tagen | Nach 21 Tagen | Nach 28 Tagen |
|---|---|---|---|---|---|---|
| Frau AL. | 4,0 | 3,5 | 3,5 | 3,5 | 4,0 | 4,0 |
| Herr BB. | 4,5 | 3,5 | 3,5 | 4,0 | 4,0 | 4,0 |
| Herr SH. | 4,3 | 3,0 | 3,5 | 4,0 | 4,0 | 4,0 |
| Durchschn itt | 4,3 | 3,3 | 3,5 | 3,8 | 4,0 | 4,0 |

**Erkennbarkeit des Gelbstiches bei Modell H**

| Mittel | 1 "nicht erkennbar" | 2 "schwer erkennbar" | 3 "leicht erkennbar" | 4 "stark sichtbar" | 5 "unübersehbar" |
|---|---|---|---|---|---|
| erfindungsgemäß en Färbung | - | SH, BB, AL. | - | - | - |
| Vergleichsfärbun g | - | - | SH, BB, AL | - | - |

**Bewertung der erfindungsgemäßen Färbung bei Modell Z**

| Friseur | Vor der Färbung | Tag der Färbung | Nach 7 Tagen | Nach 14 Tagen | Nach 21 Tagen | Nach 28 Tagen |
|---|---|---|---|---|---|---|
| Frau L. | 3,5 | 2,0 | 2,0 | 2,0 | 2,0 | 2,5 |
| Herr B. | 4,0 | 2,5 | 2,5 | 2,5 | 3,0 | 3,0 |
| Herr H. | 4,5 | 1,5 | 2,0 | 2,5 | 2,5 | 3,0 |
| Durchschn itt | 4,0 | 2,0 | 2,2 | 2,3 | 2,5 | 2,8 |

**Bewertung der Vergleichsfärbung bei Modell Z**

| | Vor der Färbung | Tag der Färbung | Nach 7 Tagen | Nach 14 Tagen | Nach 21 Tagen | Nach 28 Tagen |
|---|---|---|---|---|---|---|
| Frau AL. | 3,5 | 3,5 | 3,5 | 4,0 | 4,0 | 4,5 |
| Herr BB. | 4,0 | 3,0 | 3,0 | 3,5 | 4,0 | 4,0 |
| Herr SH. | 4,5 | 2,5 | 3,0 | 3,5 | 4,0 | 4,0 |
| Durchschn itt | 4,0 | 3,0 | 3,2 | 3,7 | 4,0 | 4,2 |

**Erkennbarkeit des Gelbstiches bei Modell Z**

| Mittel | 1 "nicht erkennbar" | 2 "schwer erkennbar" | 3 "leicht erkennbar" | 4 "stark sichtbar" | 5 "unübersehbar" |
|---|---|---|---|---|---|
| erfindungsgemäß en Färbung | - | SH, BB, AL. | - | - | - |
| Vergleichsfärbun g | - | BB, AL. | SH | - | - |

Aus diesen stichprobenartigen Ergebnissen ist klar erkennbar, dass sich sowohl die Qualität der erfindungsgemäßen Färbung als auch deren Haltbarkeit erheblich verbessert hat. Des Weiteren ist erkennbar, dass ein Gelbstich fast eliminiert wird und durch die Qualität der Färbung auch nach 4 Wochen nicht so stark wieder auftaucht.

## Patentansprüche

1. Mittel zur oxidativen Färbung von Keratinfasern, **dadurch gekennzeichnet, dass** es eine Kombination aus 1,4-Diamino-benzol (p-Phenylendiamin) oder einem p-Phenylendiaminderivat und 2,7-Naphthalendiol als alleinigen Kuppler oder deren physiologisch verträgliche, wasserlösliche Salze enthält, wobei das 1,4-Diaminobenzol (p-Phenylendiamin) oder p-Phenylendiamin-derivat ausgewählt ist aus 1,4-Diamino-benzol (p-Phenylendiamin), 1,4-Diamino-2-methyl-benzol (p-Toluyldiamin), 1,4-Diamino-2-methoxymethyl-benzol, 1,4-Diamino-2-hydroxyethyl-benzol oder N,N-Bis-(2-Hydroxyethyl)-p-phenylendiamin, oder Gemischen daraus.

2. Mittel nach Anspruch 1 **dadurch gekennzeichnet, dass** das p-Phenylendiamin-derivat und das 2,7-Naphthalenediol jeweils in einer Gesamtmenge von 0,05 bis 2 Gewichtsprozent im gebrauchsfertigen Mittel enthalten sind.

3. Mittel nach einem der Ansprüche 1 oder 2, **dadurch gekennzeichnet, dass** das p-Phenylendiamin-derivat und das 2,7-Naphthalenediol jeweils in einer Gesamtmenge von 0,2 bis 1 Gewichtsprozent im gebrauchsfertigen Mittel enthalten sind.

4. Verwendung des Mittels nach einem der Ansprüche 1 bis 3 zur Erzielung einer Graufärbung von menschlichen Haaren, deren natürliche Farbe dunkelblond bis schwarz ist und deren Anteil weißer Haare mehr als 10 % beträgt.

5. Verwendung nach Anspruch 4 wobei die Haare vor der Graufärbung noch nie gefärbt waren und daher nur Eigenpigmente des Verwenders enthalten.

6. Verwendung nach Anspruch 5 wobei die Haare vor der Graufärbung einen Gelbstich besitzen, der nach der Färbung nicht mehr erkennbar ist.

## Claims

1. An agent for oxidatively dyeing keratin fibres, **characterized in that** it contains s a combination of 1,4-diamino-benzene (p-phenylenediamine) or a p-phenylenediamine derivative and 2,7-naphthalenediol as the sole couplers or the physiologically compatible water-soluble salts thereof, wherein the 1,4-diaminobenzene (p-phenylenediamine) or p-phenylenediamine derivative is selected from 1,4-diaminobenzene (p-phenylenediamine), 1,4-diamino-2-methyl-benzene (p-toluyldiamine), 1,4-diamino-2-methoxymethyl-benzene, 1,4-diamino-2-hydroxyethyl-benzene or N,N-bis-(2-hydroxyethyl)-p-phenylenediamine, or mixtures thereof.

2. The agent according to claim 1, **characterized in that** the p-phenylenediamine derivative and the 2,7-naphthalenediol are each present in a total amount of 0.05 to 2% by weight in the ready-to-use composition.

3. Composition according to one of claims 1 or 2, **characterized in that** the p-phenylenediamine derivative and the 2,7-naphthalenediol are each present in a total amount of 0.2 to 1 % by weight in the ready-to-use composition.

4. Use of the composition according to any one of claims 1 to 3 for 1 for dyeing human hair gray, whose natural color is dark blond to black and whose proportion of white hair is more than 10%.

5. Use according to claim 4 wherein the hair has never been before dyed prior to being dyed gray and therefore only contains intrinsic pigments of the user.

6. Use according to claim 5, wherein the hairs have a yellow tinge before being dyed gray, which is no longer noticeable after being dyed.

## Revendications

1. Agent de coloration oxydative de fibres de kératine, **caractérisé en ce qu'**il contient une association d'un 1,4-diaminobenzène (p-phénylène diamine) ou d'un dérivé de p-phénylène diamine et de 2,7-naphtaline diol en tant que seul coupleur, ou leur sels hydrosolubles, physiologiquement compatibles, le 1,4-diaminobenzène (p-phénylène diamine) ou dérivé de p-phénylène diamine étant sélectionné parmi le 1,4-diaminobenzène (p-phénylène diamine), le 1,4-diamino-2-méthyl-benzène (p-toluène diamine), le 1,4-diamino-2-methoxyméthyl-benzène, le 1,4-diamino-2-hydroxyéthyl-benzène ou la n,n-bis-(2-hydroxyéthyl)-p-phénylène diamine, ou leurs mélanges.

2. Agent selon la revendication 1, **caractérisé en ce que** le dérivé de p-phénylène diamine et le 2,7-naphtaline diol sont contenus chacun dans une quantité totale de 0,05 à 2 pourcent en poids dans l'agent prêt à l'emploi.

3. Agent selon l'une quelconque des revendications 1 ou 2, **caractérisé en ce que** le dérivé de p-phénylène diamine et le 2,7-naphtaline diol sont contenus chacun dans une quantité totale de 0,2 à 1 pourcent en poids dans l'agent prêt à l'emploi.

4. Utilisation de l'agent selon l'une quelconque des revendications 1 à 3 pour obtenir une coloration grise de cheveux humains, dont la couleur naturelle est de blond foncé à noir et dont le pourcentage de cheveux blancs est supérieur à 10 %.

5. Utilisation selon la revendication 4, avant la coloration en gris, les cheveux n'ayant encore jamais été colorés et ne contenant ainsi que des propres pigments de l'utilisateur.

6. Utilisation selon la revendication 5, avant la coloration en gris, les cheveux présentant une pointe de jaune, qui n'est plus perceptible après la coloration .
